(19) Europäisches Patentamt
European Patent Office
Office européen des brevets

(11) Veröffentlichungsnummer: **0 054 897**
**B1**

(12) **EUROPÄISCHE PATENTSCHRIFT**

(45) Veröffentlichungstag der Patentschrift: **31.07.85**

(51) Int. Cl.⁴: **C 12 P 13/04**, C 07 B 57/00, C 12 P 41/00

(21) Anmeldenummer: **81110474.4**

(22) Anmeldetag: **16.12.81**

(54) **Verfahren zur enzymatischen Herstellung von L-2-Amino-4-methylphosphinobuttersäure.**

(30) Priorität: **23.12.80 DE 3048612**

(43) Veröffentlichungstag der Anmeldung: **30.06.82 Patentblatt 82/26**

(45) Bekanntmachung des Hinweises auf die Patenterteilung: **31.07.85 Patentblatt 85/31**

(84) Benannte Vertragsstaaten: **AT BE CH DE FR GB IT LI NL SE**

(56) Entgegenhaltungen:
**FR - A - 2 438 054**
**GB - A - 1 369 462**

**CHEMICAL ABSTRACTS, Band 81, Nr. 13, 30. September 1974, Seite 351, Nr. 76472y, Columbus, Ohio, USA**
**JOURNAL OF ANTIBIOTICS, Band XXXI, Nr. 8, August 1978, Seiten 783-788, Tokyo, JP, A. PLASKIE et al.: "Substrate specificity of penicillin acylase of E. Coli"**
**MICROBIOLOGY ABSTRACTS, Band 10, Nr. 3, Marz 1975, Section A, Industrial and applied microbiology, Seite 8, London, G.B.**

(73) Patentinhaber: **HOECHST AKTIENGESELLSCHAFT, Postfach 80 03 20, D-6230 Frankfurt am Main 80 (DE)**

(72) Erfinder: **Grabley, Susanne, Dr., Mörikestrasse 6, D-6233 Kelkheim (Taunus) (DE)**
Erfinder: **Sauber, Klaus, Dr., Falkenstrasse 35, D-6232 Bad Soden am Taunus (DE)**

**EP 0 054 897 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

BUNDESDRUCKEREI BERLIN

**Beschreibung**

L-2-Amino-4-methylphosphinobuttersäure (im Folgenden als L-Phosphinothricin oder L-PTC bezeichnet) oder deren Salze mit organischen oder anorganischen Basen oder Säuren sind — wie auch aus DT-OS 2 939 269 bekannt geworden ist — die wirksame Komponente der chemisch leicht zugänglichen Racemate. Letztere besitzen gemäß DT-OS 2 717 440 eine sehr gute und breite herbizide Wirksamkeit gegen zahlreiche monokotyle und dikotyle, einjährige und mehrjährige Unkräuter. Da L-PTC und seine oben angeführten Derivate gegenüber den Racematen etwa doppelt so stark wirksam ist, war es wünschenswert, ein Verfahren zu entwickeln, mit dem es möglich ist, L-PTC auf einfache Weise in größeren Mengen zugänglich zu machen.

L-PTC konnte bereits durch saure Hydrolyse (JA-OS 73-85 538) oder enzymatischen Abbau (JA-OS 74-31 890) L-PTC-alanylalanin, einem literaturbekannten mikrobiell gewonnenen Antibiotikum erhalten werden.

In der DT-OS 2 939 269 ist ferner ein Verfahren beschrieben, bei dem N-Acyl (insbesondere N-Acetyl)-L-PTC mittels mikrobieller Acylasen, die in Form ganzer Zellen oder Zellextrakte eingesetzt wurden und aus speziell gezüchteten Stämmen der Gattung Pseudomonas, Streptomyces oder Aspergillus stammen, schneller gespalten wird als das entsprechende D-Derivat. Die verwendeten Acylasen besitzen gemäß den Angaben der DT-OS kaum eine oder nur eine sehr geringe Wirkung gegenüber anderen Substraten als N-Acyl-L-PTC, beispielsweise gegenüber N-Acylderivaten von bekannten üblichen L-Aminosäuren. Das nach der Aufarbeitung isolierte PTC hat zudem nur einen maximalen spez. Drehwert $[\alpha]_D^{22}$ von 23° (c = l, 1 N HCl), was einer optischen Reinheit von nur etwa 75% entspricht.

Untersuchungen haben jedoch gezeigt, daß handelsübliche Acylasen wie Acylase I (Aminoacylase aus Schweinenieren; EC 3.5.1.14) oder mikrobielle AMANO-Acylase, welche bei natürlichen Aminosäuren gut zur Racematspaltung geeignet sind, keinerlei Aktivität bei D,L-Acyl-PTC zeigen. Dies bestätigt die Angaben aus der DT-OS 2 939 269, wonach zur fermentativen Herstellung PTC-aktiver Acylasen ein aufwendiges mikrobiologisches Screening notwendig ist. Auch andere Enzyme, z. B. proteolytische Enzyme mit Esteraseaktivität, welche bei herkömmlichen D,L-Aminosäuren hohe Aktivität und Selektivität aufweisen, besitzen bei D,L-PTC-Estern keine oder nur eine stark verminderte Aktivität.

Überraschenderweise wurde nun gefunden, daß Penicillin-G-Acylase phenacetyliertes D,L-PTC nicht nur mit ungewöhnlich hoher Reaktionsgeschwindigkeit, die derjenigen bei der Deacylierung von natürlichem Penicillin G nahekommt, deacyliert, sondern auch eine im Vergleich zu den in DT-OS 2 939 269 beschriebenen mikrobiellen Acylasen erheblich gesteigerte Selektivität besitzt, die eine außerordentlich hohe Reinheit des gebildeten L-PTC zur Folge hat.

Die Erfindung betrifft infolgedessen ein Verfahren zur enzymatischen Trennung von D,L-2-Amino-4-methylphosphinobuttersäure, welches dadurch gekennzeichnet ist, daß man deren N-Aracetyl-Derivate der Formel

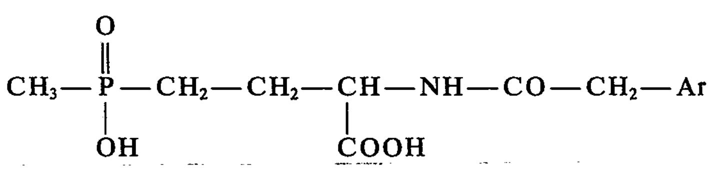

(I)

(in welcher Ar einen gegebenenfalls durch Halogen oder Hydroxy substituierten Phenylrest darstellt) in wäßrigem oder wäßrig-organischem Medium mit einer Penicillin-G-Acylase behandelt.

Zwar ist bekannt, daß an bestimmten phenacetylierten herkömmlichen D,L-Aminosäuren mit aus E. coli gewonnener Penicillinacylase eine selektive Hydrolyse zu freier L-Aminosäure möglich ist (GB-PS 1 369 462; Bioorganiçeskaja Khimia 5 (1979), 604 ff.). Es ist aber auch bekannt, daß Variationen am Aminosäureteil des Substrats zu einem starken Abfall der enzymatischen Aktivität der Penicillin-G-acylase führen (A. Plaskie et al., J. Antibiotics 31, 783 (1978)). Es war daher nicht zu erwarten, daß mittels der erfindungsgemäß verwendeten Penicillin-G-acylasen L-PTC in hoher Reinheit erhalten werden kann.

Bei dem erfindungsgemäßen Verfahren fällt ein Gemisch aus L-PTC und N-Phenacetyl-D-PTC sowie Phenylessigsäure an, aus dem das L-PTC leicht in bekannter Weise abgetrennt werden kann. Beispielsweise kann — gegebenenfalls nach Filtration — die wäßrige Reaktionslösung durch eine Kolonne geleitet werden, die mit einem stark sauren Kationenaustauscherharz in der $H^{(+)}$-Form beladen ist. Während N-Phenacetyl-D-PTC und Phenylessigsäure die Kolonne passieren, wird das L-PTC am Harz adsorbiert. Die Elution kann dann in bekannter Weise mit verdünnter Salzsäure oder Ammoniak vorgenommen werden. Das Eluat kann gegebenenfalls mit Aktivkohle gereinigt und der Gefriertrocknung unterworfen werden oder das eingeengte Eluat kann zur Kristallisation gebracht werden.

Ausgangsstoffe der Formel I erhält man in an sich bekannter Weise z. B. durch Umsetzung des Dinatriumsalzes des D,L-PTC mit Phenacetylchlorid bei −5° C bis +5° C unter gleichzeitiger Zugabe von äquimolaren Mengen Natronlauge. Das Reaktionsgemisch kann direkt der Spaltung unterworfen werden; gewünschtenfalls kann man das N-Phenacetyl-D,L-PTC auch in kristalliner Form durch Ansäuern mit Salzsäure oder Schwefelsäure gewinnen. Das enzymatisch nicht gespaltene N-Phenacetyl-D-PTC kann nach Abtrennen der Phenylessigsäure mittels Ether oder Dichlormethan durch Hydrolyse in verdünnter Salzsäure bei 80° C in D-PTC verwandelt und dieses nach Racemisierung erneut der enzymatischen Spaltung unterworfen werden.

Unter Penicillin-acylasen oder -amidasen werden solche Enzyme verstanden, die Penicillin zu 6-Amino-penicillansäure spalten können. Die für das erfindungsgemäße Verfahren geeigneten Penicillin-G-acylasen werden von prokaryonten Mikroorganismen wie Escherichia coli, Bacillus megatherium u. a. gebildet (M. Cole et al., Meth. Enzym. 43, 698 (1975)) und sind unter der E. C. Nr. 3.5.1.11 bekannt. Besonders bevorzugt ist Penicillin-G-amidase aus E. coli ATCC 11105.

Die erfindungsgemäß verwendete Penicillin-G-acylase kann als freies, wasserlösliches Lyophilisat oder in wasserunlöslicher Form an einen Träger gebunden (vgl. DT-OS 2 732 301) in einer wäßrigen Lösung eingesetzt werden, die eine Substratkonzentration zwischen 0,1 und 20%, bevorzugt 4 bis 6%, aufweisen kann. Die Reaktionstemperatur liegt zwischen 10° C und 60° C, bevorzugt zwischen 20° C und 40° C, die Reaktionsdauer beträgt je nach Substrat- und Enzymkonzentration bzw. Enzymaktivität 1—48 h. Ausreichende enzymatische Aktivität kann bei einem pH von 3—9, bevorzugt bei 6—8, beobachtet werden. Die Reaktion kann in einem Phosphatpuffer oder auch ohne Pufferzusatz durchgeführt werden. Die Reaktion mit trägergebundenem Enzym kann im Batch- oder Säulenverfahren durchgeführt werden. Im Säulenverfahren wird über das fixierte Enzym als stationäre Phase in der Säule kontinuierlich eine Substratlösung bis zur vollständigen Deacylierung des N-Aracetyl-L-PTC geleitet.

Verlauf und Ende der Reaktion können mittels polarimetrischer Methoden oder der literaturbekannten quantitativen Analysen der gebildeten freien Aminosäure durch Umsetzung mit Ninhydrin und spektralphotometrische Gehaltsbestimmung verfolgt werden.

Das in hoher Ausbeute erhaltene L-PTC hat einen Drehwert $[\alpha]_D^{22} = +28{,}5°$ (c=1 in 1 N HCl), was einer optischen Reinheit von mindestens 90% (entspr. 95% L-Form entspricht.

Die im Folgenden aufgeführten Beispiele dienen der weiteren Erläuterung der Erfindung.

## Beispiel 1

10 g (33,4 m mol) N-Phenacetyl-D,L-PTC wurden in wenig bidest. Wasser suspendiert, mit 1 N NaOH auf pH 7,8 gestellt und die Lösung mit bidest. Wasser auf ein Volumen von 500 ml aufgefüllt. Nach Zugabe von 15 mg Penicillin-G-acylase aus E. coli (Aktivität ca. 2.9 U/mg; Substrat Penicillin-G-Kaliumsalz, Reaktionstemperatur 37° C) wurde die Reaktionsmischung bei Raumtemperatur stehen gelassen. Nach 15 h wurde durch Bestimmung des Gehaltes an freier Aminosäure durch Anfärben einer Probe mit Ninhydrin ein ca. 50%iger Umsatz gefunden. Nach Ansäuern mit conc. Salzsäure auf einen pH von 2—3 und Filtration wurde die klare, wäßrige Substratlösung durch eine Kolonne geleitet, die mit 150 g Dowex® 50 W x 2 ($H^+$-Form) beschickt war. Es wurde mit Wasser nachgewaschen bis das Eluat neutral war und keine Chloridionen mehr nachzuweisen waren. Anschließend ließ sich die freie Aminosäure mit 0,8 N Salzsäure in Ethanol/Wasser 80 : 20 als Hydrochlorid eluieren. Aus dem eingeengten Eluat wurde reines PTC-hydrochlorid mit einem Schmelzpunkt von 199—200° C erhalten. Ausbeute 3,3 g (15,2 m mol ≙ 45,4%). Aus dem Hydrochlorid wurde in üblicher Weise durch Zugabe der ca. doppelten molaren Menge an Propylenoxid zur ethanolischen Substratlösung die freie kristalline L-Aminosäure isoliert: Fp. 217—219° C, $[\alpha]_D^{22} = +28°$ (C=1, 1 N HCl).

## Beispiel 2

60 g (200,5 m mol) N-Phenacetyl-D,L-PTC wurden in wenig Wasser suspendiert, mit Natronlauge auf einen pH von 8,0 gestellt, mit Wasser auf 1 l aufgefüllt und mit 6 g fixierter Penicillin-G-acylase versetzt (Aktivität ca. 80 U/g, Substrat Penicillin-G-Kaliumsalz, Reaktionstemperatur 37° C; Herstellung des Träger DT-OS 2 732 301). Nach 1,5 d Rühren bei Raumtemperatur wurde die Substratlösung vom wasserunlöslichen Katalysator abfiltriert und das Filtrat über eine Kolonne, die mit 750 g stark saurem Kationenaustauscherharz gefüllt war, geleitet und analog zu der in Beispiel 1 verwendeten Reaktionslösung weiter aufgearbeitet. Es wurden 20 g (92 m mol ≙ 46%) L-PTC-hydrochlorid mit einem Schmelzpunkt von 199—201° C erhalten. Der spezifische Drehwinkel $[\alpha]_D^{22}$ wurde zu 23,3° bestimmt (C=0,6, 1 N HCl) entsprechend einem molaren Drehvermögen von $[M]_D^{22} = 50{,}7$. Das nach Behandeln mit Propylenoxid erhaltene freie L-PTC besaß einen Schmelzpunkt von 216—217° C und einen spezifischen Drehwinkel von $[\alpha]_D^{22} = +28{,}5°$ (C=1, 1 N HCl) entsprechend einem molaren Drehvermögen $[M]_D^{22}$ von 51,6°.

## Beispiel 3

Um die Stabilität der in Beispiel 2 angewendeten trägergebundenen Penicillin-G-acylase gegenüber N-Phenacetyl-D,L-PTC zu prüfen, wurden 100 mg des fixierten Enzyms mit 10 ml einer 5%igen wäßrigen Lösung von N-Phenacetyl-D,L-PTC (pH 7,8, stabilisiert durch Zusatz von wenig Phosphatpuffer)

versetzt und bei Raumtemperatur gerührt. Nach jeweils 24 h wurde die Substratlösung abfiltriert und das abgetrennte wasserunlösliche Enzym erneut mit racemischer Substratlösung beschickt. Die Enzymaktivität wurde durch Bestimmung des Gehalts an freiem PTC nach 45 min und nach 22,5 h durch Anfärben einer Probe mit Ninhydrin ermittelt.

Nach 8 Wochen konnte keine signifikante Verminderung des PTC-Anteils nach 45 min Reaktionsdauer ermittelt werden (etwa 20% bezogen auf eingesetztes Racemat). Nach 22,5 h waren in jedem Fall 50% des eingesetzten Racemates hydrolysiert.

## Patentansprüche

1. Verfahren zur enzymatischen Trennung von D,L-2-Amino-4-methylphosphinobuttersäure, dadurch gekennzeichnet, daß man deren N-Aracetylderivate der Formel

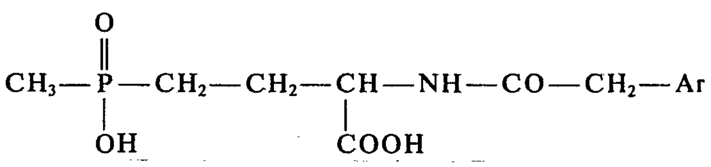

(I)

in wäßrigem oder wäßrig-organischem Medium mit einer Penicillin-G-acylase behandelt.

2. Verfahren nach Anspruch 1, dadurch gekennzeichnet, daß man Penicillin-G-acylase aus E. coli verwendet.

3. Verfahren nach Anspruch 1 und 2, dadurch gekennzeichnet, daß man Penicillin-G-acylase aus E. coli ATCC 11105 verwendet.

4. Verfahren nach Anspruch 1 bis 3, dadurch gekennzeichnet, daß die Penicillin-G-acylase als freies Lyophilisat vorliegt.

5. Verfahren nach Anspruch 1 bis 3, dadurch gekennzeichnet, daß die Penicillin-G-acylase an einen Träger gebunden ist.

## Claims

1. A process for the enzymatic separation of D,L-2-amino-4-methylphosphinobutyric acid, which comprises treating its N-aracetyl derivatives of the formula

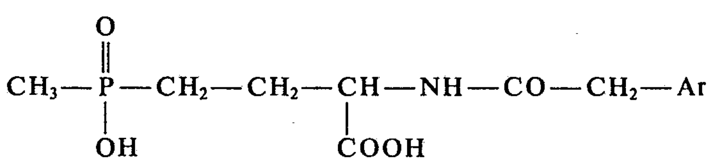

(I)

in an aqueous or aqueous-organic medium with a penicillin-G-acylase.

2. A process as claimed in claim 1, wherein penicillin-G-acylase form E. coli is employed.

3. A process as claimed in any of claims 1 and 2, wherein penicillin-G-acylase from E. coli ATCC 11,105 is employed.

4. A process as claimed in any of claims 1 to 3 wherein the penicillin-G-acylase is in the form of a free lyophilisate.

5. A process as claimed in any of claims 1 to 3, wherein the penicillin-G-acylase is bound to a carrier.

## Revendications

1. Procédé pour la séparation enzymatique d'acide D,L-amino-2 méthyl-4 phosphinobutyrique, caractérisé en ce qu'on traite ses dérivés N-aracétyle de formule

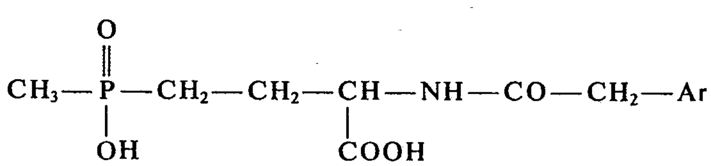

(I)

en milieu aqueux ou aqueux-organique, avec une pénicilline-G-acylase.

2. Procédé selon la revendication 1, caractérisé en ce qu'on utilise une pénicilline-G-acylase provenant de E. coli.

3. Procédé selon l'une quelconque des revendications 1 ou 2, caractérisé en ce qu'on utilise une pénicilline-G-acylase provenant de E. coli ATCC 11105.

4. Procédé selon l'une quelconque des revendications 1 à 3, caractérisé en ce que la pénicilline-G-acylase se présente sous la forme d'un lyophilisat libre.

5. Procédé selon l'une quelconque des revendications 1 à 3, caractérisé en ce que la pénicilline-G-acylase est fixée sur un support.